# EUROPEAN PATENT SPECIFICATION

(11) **EP 4 611 862 B1**
(45) Date of publication and mention of the grant of the patent: **09.12.2026**
(21) Application number: 23804944.9
(22) Date of filing: 02.11.2023
(51) Int. Cl.: A61M 16/00, F04D 25/08, F04D 29/10, F04D 29/42

(54) **DEVICE FOR PROTECTING TURBINE SHAFT BEARINGS USED IN ANAESTHESIA DEVICES FROM CONTAMINANTS IN PATIENT GASES**
VORRICHTUNG ZUM SCHUTZ VON IN ANÄSTHESIEGERÄTEN VERWENDETEN TURBINENWELLENLAGERN VOR VERUNREINIGUNGEN IN PATIENTENGASEN
DISPOSITIF DE PROTECTION DE PALIERS D'ARBRE DE TURBINE UTILISÉS DANS DES APPAREILS D'ANESTHÉSIE CONTRE LES CONTAMINANTS PRÉSENTS DANS LES GAZ DESTINÉS AU PATIENT

(30) Priority: 03.11.2022 GB 202216366; 15.03.2023 GB 202303766
(43) Date of publication of application: 10.09.2025
(73) Proprietor: Peninsula Medical Technologies Ltd, Totnes, Devon TQ9 5BN (GB)
(72) Inventor: BROWN, Sebastian, Devon TQ9 5BN (GB)
(74) Representative: Bryers Intellectual Property Ltd
(86) International application number: PCT/EP2023/080616
(87) International publication number: WO 2024/094824

(56) References cited:
- EP-B1- 2 122 180
- WO-A1-2017/039497
- CN-A- 106 151 097
- FR-A1- 2 910 078
- FR-A3- 3 035 456

## Description

Modern ventilation systems used to replace or assist breathing function for patients undergoing surgery or who are unable to breathe normally for themselves due to illness often use turbines ("blowers") for the delivery of pressure to the patient. Turbines consist of a motor, impeller, gas casing and heatsink. Gas is drawn in through the inlet and passes out through the outlet. There are different configurations of turbine (single stage, dual stage etc.) and the turbine can be used in different ways; highly dynamic turbines can speed up and down in sequence with the breath and less dynamic turbines may provide a high pressure to a valve that allows gas in at the desired flow rate for the breath.

The present disclosure refers in part to the use of turbines in a modular anaesthesia and ventilation system described in WO 2022/049389. In this system, a portable ventilator module provides ventilation for patients in transport, intensive care, wards and long-term ventilation facilities. Ventilation can be invasive ventilation, non-invasive ventilation or the provision of high flow oxygen in mandatory, spontaneous or mixed modes as familiar to those skilled in the art. When working alone, the ventilation module mixes pressurised oxygen with ambient air drawn into the device by the turbine as determined by the user. This gas mixture is then delivered to the patient during the inspiratory phase and subsequently exhaled, through a valve that maintains the circuit pressure, to atmosphere. At each point in the circuit, additional components are required to monitor and control pressure, temperature and flow.

The portable ventilation module can be reversibly connected ("docked") to an anaesthesia module to provide functionality required for anaesthesia. This includes the addition and recirculation of anaesthetic agent (volatile anaesthetics such as Sevoflurane, Desflurane or Isoflurane or gases such as xenon or nitrous oxide), addition of oxygen/air, removal of carbon dioxide, provision for manual ventilation, exhaust and waste gas scavenging systems. Other systems may be included to control temperature and/or humidity.

As anaesthesia systems recirculate exhaled patient gas, these systems must be suitable for cleaning/sterilisation as required and compatible with anaesthetic agents/gases used to induce or maintain anaesthesia. These anaesthesia gases (as mentioned above) are effective solvents for many standard lubricants or incompatible with often-used seals (e.g. FKM). Due to the high speed of rotation of the turbine (e.g. 60,000rpm) and dynamic nature (the turbine may accelerate and decelerate up to 100 times per minute for each breath), it is challenging to seal the turbine shaft to prevent exposure of the shaft bearings and lubricant to gases passing through the turbine. These gases contain anaesthetic vapour and microbiological contamination from exhaled patient gases and can affect the long-term performance of the turbine by degrading the lubricant or contaminate the turbine bearings/shaft which may then not sterilisable.

Solutions that may remove the impeller from the turbine motor for cleaning/sterilisation are not favourable as the high impeller rotational speed needs very precise balancing after manufacture. Removal and replacement may affect this balance leading to damage to the turbine motor and degradation in performance during the operation of the turbine.

Other systems are described in FR 3 035 456 A3, FR 2 910 078 A1, EP 2 122 180 B1 and WO 2017/039497 A1.

The present disclosure relates to apparatus and methods to prevent the impairment of performance of lubricants used in the motor by exposure to volatile anaesthetic gases used in anaesthesia. This may be provided by a modular system as mentioned above or by conventional anaesthesia systems incorporating a turbine.

### Summary of the invention:

The present invention provides a turbine for the delivery of gas to a patient as defined in claim 1. Further preferred embodiments of the present invention are defined in the dependent claims.

### Summary of the present disclosure:

Reference will now be made to various exemplary embodiments or aspects of the present disclosure. Embodiments or aspects disclosed herein which do not fall under the scope of the appended claims do not form part of the present invention, but are useful for understanding the principles of the invention. The scope of the present invention is defined by the appended claims.

The present disclosure provides or relates to apparatus resulting in the protection of turbine shaft lubricants and components (e.g. bearings) from degradation induced by anaesthesia gases (Sevoflurane, Desflurane, Isoflurane, Nitrous Oxide and Xenon) by using a flush gas flow of pure oxygen through a gas collar surrounding the turbine shaft.

The gas collar serves to deliver the oxygen gas to the shaft and reduce the outward flow of oxygen gas to maintain a minimal gas flush flow rate. As oxygen is always required by the patient as a basal metabolic rate and a basal flow of oxygen is recommended/required by standards/convention, this gas flush does not generate additional wastage of gas or impair the delivery of anaesthesia, especially when anaesthesia gas injection systems are used familiar to those skilled in the art.

A gas channel is made from the outside of the turbine to the motor-end of the shaft. A connection is made at the external end of this gas channel for the connection of the oxygen supply.

A collar may be secured to the turbine housing, around the shaft. This collar may be a separate component sealed to the turbine housing as a separate component or integrated as part of the turbine housing design. The collar serves to distribute gas from the channel around the base of the motor shaft. This gas then passes around the shaft and underneath the impeller to join the gas from the breathing circuit.

In this way, at outward flow of gas from the shaft is provided to prevent patient gas containing anaesthesia gas and contaminants from surrounding the bearing and interfering with the function of the bearing.

In one embodiment, the collar is a separate piece that is secured to the motor or turbine housing by screws and may contain a seal to prevent gas from flowing between the collar and the housing.

In another embodiment, the turbine housing is built with the collar integrated into the housing.

In one embodiment, the collar is made of metal, e.g. aluminium or stainless steel or another suitable metal familiar to those skilled in the art.

In another embodiment, the collar is made of plastic such as nylon, polyetherimide (PEI) or PEEK or another suitable plastic familiar to those skilled in the art.

In one embodiment as shown in the drawings, the collar does not extend to the full diameter of the impeller.

In another embodiment, the collar extends to the full diameter of the impeller.

In one embodiment, there is a single gas channel.

In another embodiment, there are multiple gas channels.

In another embodiment, the impeller-side of the collar has a surface with recessed circles of increasing diameter to trap gas and form a gas-seal with the underside of the impeller.

In a preferred embodiment, the internal surface of the collar has grooves machined into the face that is in proximity to the shaft that are filled with flush gas and operate as a gas bearing on the shaft, preventing ingress of gases contaminated with anaesthetic vapours or exhaled from the patient (and containing microbiological contaminants).

In one embodiment of the disclosure, a plate is recessed into the heatsink to maintain a flat surface and acts as a distributor of gas from the oxygen connection source to the motor shaft. The plate is secured by screws and sealed at the distal edge by o-ring seal to direct gas to the motor shaft, although other methods of securing and sealing such as adhesive or sealant are envisaged.

In one embodiment of the disclosure, a gas collar is part of the distributor plate to further protect the motor shaft from the external volatile anaesthetic agent.

In one embodiment of the disclosure, the flow rate of flush gas is varied according to the concentration and type of volatile anaesthetic agent being used. The volatile anaesthetic agents may be used in different concentrations depending on the depth of anaesthesia required or the type of anaesthetic in use. Thus, to achieve the same depth of anaesthesia, Desflurane needs to be given at concentrations of around 6% (one Minimum Alveolar Concentration- MAC) and Sevoflurane at 2%. In one embodiment of the disclosure, the flush gas rate is increased when used with higher concentrations of anaesthetic gas.

In another embodiment of the disclosure, the flush gas rate is controlled by a computer model based on the concentration and type of anaesthetic gas.

In a further embodiment of the disclosure, the flush gas rate is controlled by a computer model based on the concentration and/or type of anaesthetic gas and/or rotational speed of the turbine.

Activated carbon is an effective absorbent of volatile anaesthetic agents. Depending on the carbon source, pore size and dopants, the binding affinity can be adjusted. The use of a flush gas will drive absorbed volatile anaesthetic from the activated carbon.

In an embodiment of the disclosure, a replaceable activated carbon collar is placed around the motor shaft. This activated carbon collar provides a physical partial barrier to the passage of anaesthetic gases to the motor shaft. It also absorbs any volatile anaesthetic agent near the motor shaft. Due to the presence of the flush gas which is delivered to the motor side of the activated carbon and flows around/through the activated carbon to pass to the turbine outlet, any absorbed volatile anaesthetic is desorbed from the activated carbon, further protecting the bearings/lubricants from degradation by exposure to volatile anaesthetic gases.

In an embodiment of the disclosure, the flush gas may be pure oxygen delivered to the turbine within the machine. This may be medical oxygen in the case of a medical device. This may be medical air if oxygen is not required. This may be a mixture of medical oxygen/medical air as required.

In an embodiment of the disclosure, the flush gas may be recycled from the patient circuit. Patient gas is withdrawn from the patient circuit by a pump, as familiar to those skilled in the art. The flush gas may then be scrubbed of moisture by the use of absorbent materials (e.g. silica gel) or a membrane (e.g. Nafion) and. The gas is then scrubbed of volatile anaesthetic agent by a carbon filter before being passed back into the turbine as flush gas with no/low concentration of volatile anaesthetic gas.

In one embodiment of the disclosure, the moisture scrubbing absorbent is replaceable.

In another embodiment of the disclosure, the carbon filter is replaceable.

In an embodiment of the disclosure, two carbon filters are used. In one state, patient gas is passed into a first carbon filter where volatile anaesthetic is absorbed onto the filter and then to the turbine as a flush gas. At the same time, fresh oxygen and/or air is passed through a second carbon filter to remove absorbed volatile anaesthetic, regenerate the filter, and return the anaesthetic to the anaesthesia circuit. When the first carbon filter is nearly saturated, the state is changed so that the first carbon filter is exposed to fresh oxygen and/or air to remove absorbed volatile anaesthetic agent, regenerate the filter, and return the volatile anaesthetic agent to the patient circuit and patient gas is passed into the second carbon filter to remove volatile anaesthetic agent, which is then used as a flush gas for the turbine.

In one embodiment of the disclosure, the filter material may be one of activated carbon, silica, halogenated carbon, carbon, graphite, aerogel (carbon or silica-based) or other filter materials known to reversibly absorb volatile anaesthestics/fluorocarbons to those skilled in the art.

In one embodiment of the disclosure, the canister may be heated or exposed to vacuum during regeneration to improve the desorption of volatile anaesthetic.

In one embodiment of the disclosure, water may be removed before the absorption of volatile anaesthetic, by the use of a preceding water-absorbent filter such as silica gel. Due to the presence of exhaled patient gas in circle systems used for anaesthesia gas delivery and efficiency, the flush gas also prevents contamination of the turbine shaft/bearings with exhaled patient gas which may contain chemical or microbial contaminants (e.g. viruses/spores/bacteria etc). The presence of exhaled patient gas in the circuit means that the areas of the turbine exposed to patient gas must be capable of sterilisation or disinfection.

In one embodiment of the disclosure, the impeller housing is detachable from the turbine heat sink, motor, impeller and flush gas mechanism. The impeller housing can then be taken away by the user for sterilisation (e.g. autoclave although other methods as mentioned below or known to those skilled in the art can be used) and replaced when cleaned. By only removing the impeller housing, the flush gas mechanism can remain connected to the turbine. As this is a gas connection, it is preferable to leave this connection intact rather than it being repeatedly broken and re-made for each sterilisation cycle.

In an embodiment of the disclosure, the impeller housing may be replaced by another housing that is able to administer a sterilising agent to the remaining components of the turbine so that these may also be sterilised or disinfected in situ. Such sterilising agents may include but are not limited to ethylene oxide, hydrogen peroxide (vapourised or plasma) or chlorine dioxide although other methods are familiar to those skilled in the art.

In one embodiment of the disclosure, the replacement housing for sterilising the remaining parts of the turbine introduces a source of UVC radiation that is configured to be delivered to all parts of the turbine that are exposed to exhaled patient gas.

In a preferred embodiment, the use of the flush gas delivered to the shaft of the turbine means that this is not exposed to exhaled patient gas and only the parts after the exit of flush gas from the collar are considered exposed, and these are accessible to the UVC radiation for sterilisation.

In another embodiment of the disclosure, other direct sources of sterilisation may be used in place of or in addition to UVC radiation. These include gamma irradiation and electron beam irradiation, although other methods may be used familiar to those skilled in the art.

Each means of sterilisation can be controlled by a device attached to or as part of the sterilising impeller housing or the sterilisation process may be controlled by the ventilator module itself following electrical (communications and/or power) connection to the sterilising impeller housing.

Different aspects and embodiments of the disclosure may be used separately or together.

Further particular and preferred aspects of the present invention are set out in the accompanying independent and dependent claims.

The present disclosure is now more particularly described, by way of example, with reference to the accompanying drawings.

Example embodiments are described in sufficient detail to enable those of ordinary skill in the art to embody and implement the systems and processes herein described. It is important to understand that embodiments can be provided in many alternate forms and should not be construed as limited to the examples set forth herein.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

### Description of drawings:

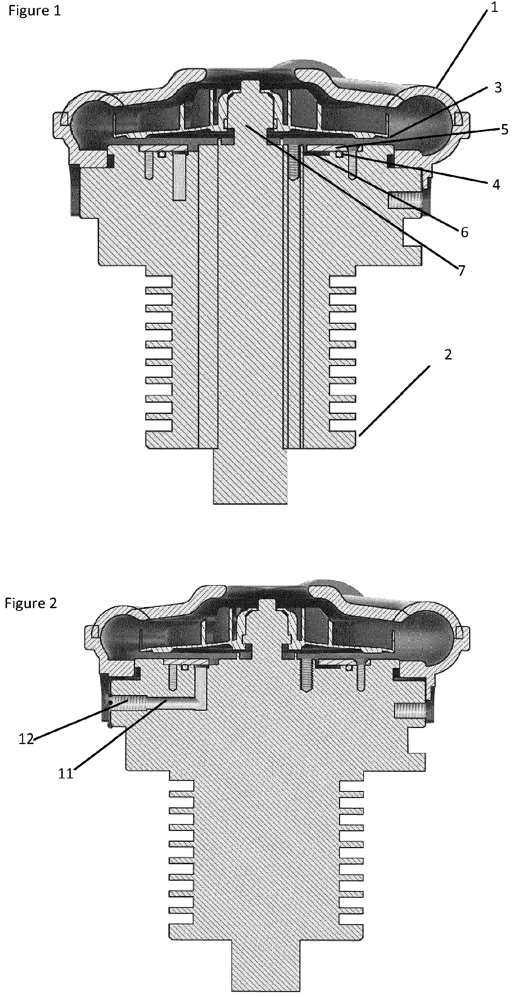

Figure 1 shows the turbine heatsink 2, cover 1, impeller 3 and motor 7 in cross section. A plate 5 is secured to the heatsink with screws. The plate conceals a channel 6 for gas to flow towards the motor spindle 7. Flush gas is contained at the outer edge by an o-ring seal 4 to ensure that gas exits the channel 6 by the motor spindle 7.
Figure 2 shows the channel through the heatsink 11 that passes flush gas to the channel 6 in Figure 1. A thread 12 may be cut into the channel to secure a connection (not shown) to the heatsink.

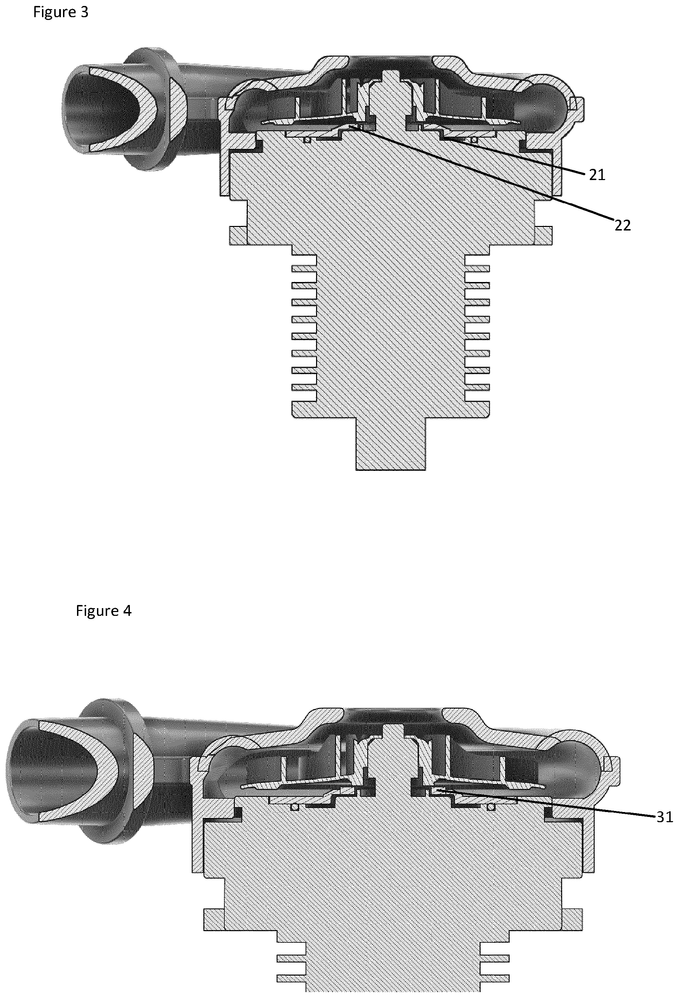

Figure 3 shows the plate with a raised collar 22 that comes in close proximity to the impeller base to further direct gas to the spindle of the motor through the channel 21.
Figure 4 shows an activated carbon filter 31 in close proximity to the impeller. The carbon filter rests on the heatsink but has ridges/channels on the underside to allow the passage of gas towards the spindle.

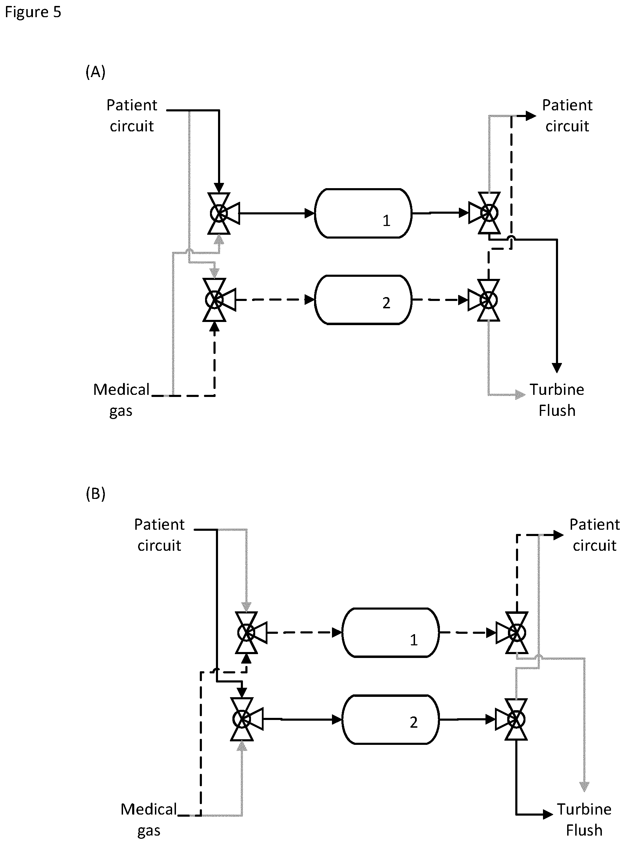

Figure 5 shows a 1^{st} and 2^{nd} activated charcoal canister connected to patient circuit and medical gas inlets and to patient circuit and turbine flush outlets. At any stage, by the use of 3 port, 2 position valves, one carbon canister is scrubbing gas from the patient circuit and one carbon canister is being regenerated by a flow of clean medical gas through the canister and back into the patient circuit.
In (5A), canister 1, filled with absorbent, is used to scrub patient gases for use as the turbine flush while canister 2 may be emptied of anaesthetic by the medical gas flush back into the patient circuit to prevent loss of anaesthetic.
In (5B), canister 2 is used to scrub patient gases for use as the turbine flush and canister 1, as this is now full of absorbed anaesthetic, is emptied of anaesthetic back into the patient circuit.
   It is envisaged that a circuit configuration can include adsorption and desorption steps with gas flow in the opposite direction to improve efficiency.

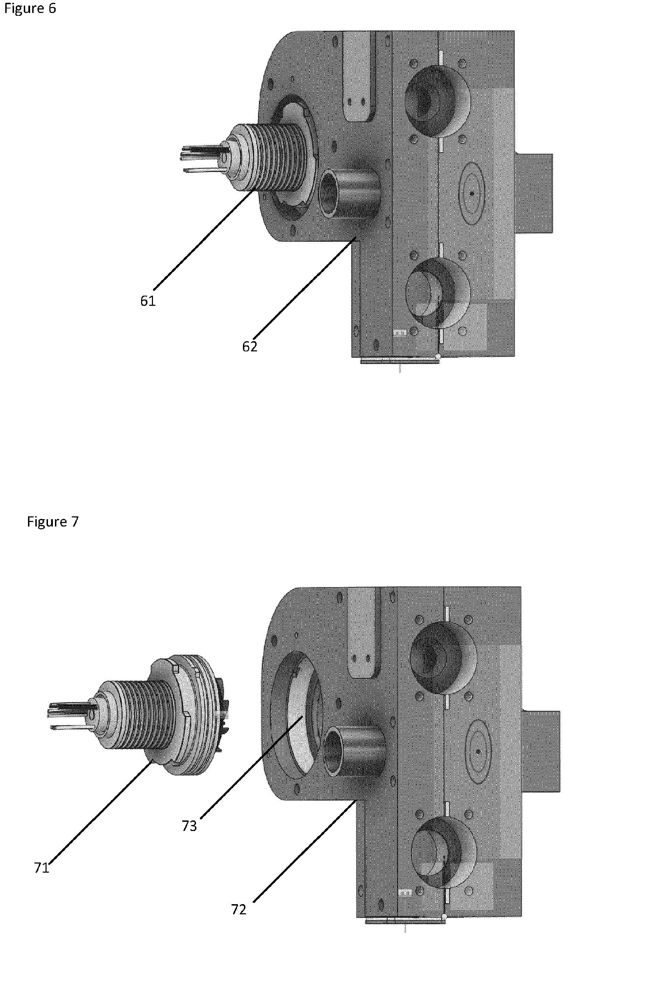

Figure 6 shows the turbine 61 engaged into the patient circuit manifold 62.
Figure 7 shows the turbine heat sink, motor and impeller 71 separated from the patient circuit manifold 72. The turbine heat sink, motor and impeller are left behind in the device and remain electrically connected and connected to the flush gas source in the device. The patient circuit manifold, which includes the impeller housing 73 can then be taken away for disassembly and sterilisation while the turbine heat sink, motor and impeller remain for in-situ sterilisation as described in the description.

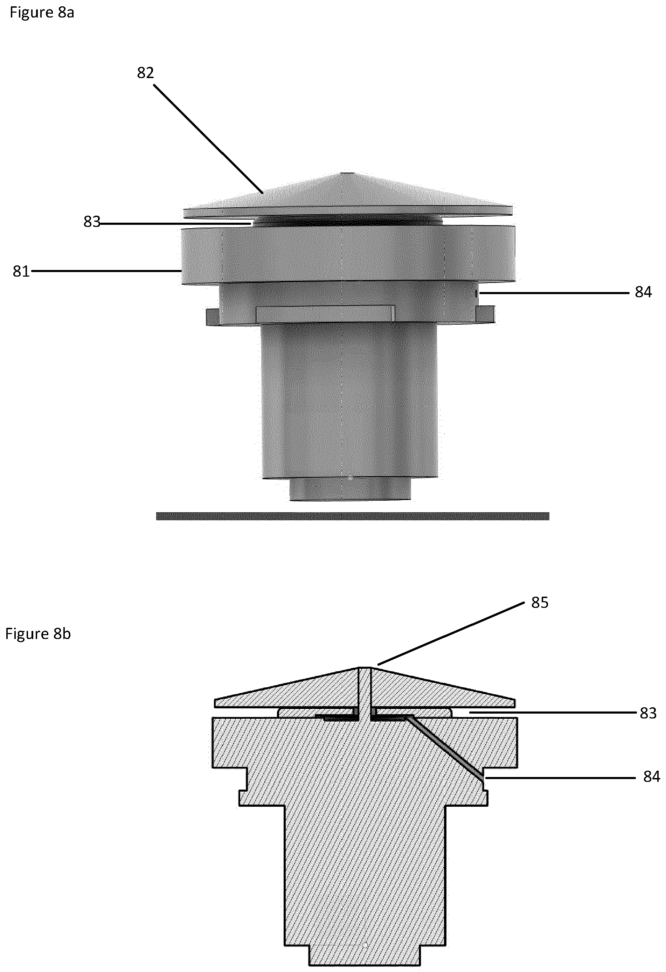

Figure 8a/8b are a ventilation turbine without the impeller housing/cover, showing the channel and collar for the supply of flush gas in side view (8a) and mid-section (8b). The heatsink and motor 81 are attached to the collar 83 which supplies flush gas from the gas inlet port 84 to the base of the shaft 85 to the impeller 82. This collar directs the gas around the shaft to protect the bearings from contamination, with gas flowing radially outwards from the shaft 85, under the impeller 82 to the outlet of the turbine (not shown). This prevents contamination of the turbine bearings with anaesthetic gases or exhaled patient gases.

Although illustrative embodiments of the disclosure have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims.

## Claims

1. A turbine for the delivery of gas to a patient, the turbine comprises a motor (7), an impeller (3, 71) and a housing including a motor shaft (85) having a motor end, the turbine further comprises a gas channel (6) to the motor-end of the shaft, whereby flushing gas can be delivered to the region of the base of the motor shaft and distributed to prevent exposure of the motor to patient gases, in which a collar (22, 83) is configured to deliver the flushing gas to the shaft and reduce the outward flow of oxygen gas to maintain a minimal gas flush flow rate, **characterised in that** a motor shaft surface of the collar is in close proximity to the motor shaft and has recessed circles in the collar to trap gas and form a gas-bearing seal with the motor shaft, or **in that** an impeller-side of the collar has a surface with recessed circles of increasing diameter to trap flushing gas and form a gas-seal with the underside of the impeller.

2. A turbine as claimed in claim 1, in which flushing gas passes between the collar and shaft towards an impeller end of the shaft to maintain a minimal gas flush flow rate.

3. A turbine as claimed in any preceding claim, whereby a connection can be made at the external end of the gas channel for the connection of flushing gas supply.

4. A turbine as claimed in any preceding claim, in which the collar is a separate component that is secured to the turbine housing, around the shaft.

5. A turbine as claimed in any of claims 1 to 3, in which the collar is integrated as part of the turbine housing.

6. A turbine as claimed in any preceding claim, in which the collar is configured such that flushing gas then passes around the shaft and underneath the impeller to join gas from a breathing circuit.

7. A turbine as claimed in any preceding claim, in which the collar does not extend to the full diameter of the impeller, or in which the collar extends to the full diameter of an impeller.

8. A turbine as claimed in any preceding claim, in which the collar has a single gas channel or multiple gas channels.

9. A turbine as claimed in any preceding claim which has a removable impeller housing (73) that can be sent for sterilisation and wherein the turbine housing, motor and impeller remain in the device to maintain electrical and flush gas connections.

10. A turbine as claimed in claim 9, wherein the removable impeller housing (73) can be replaced by a sterilisation device that delivers sterilising fluids, vapours or radiation to the turbine housing, motor and impeller to sterilise the parts of the turbine remaining in the device after the impeller housing is removed.

11. A turbine as claimed in any preceding claim, in which the flushing gas is medical air and/or medical oxygen.

12. A patient anaesthesia device having a turbine as claimed in any preceding claim.

13. A patient ventilator having a turbine as claimed in any of claims 1 to 11.

## Patentansprüche

1. Turbine für die Zufuhr von Gas zu einem Patienten, wobei die Turbine einen Motor (7), ein Laufrad (3, 71) und ein Gehäuse umfasst, das eine Motorwelle (85) mit einem Motorende enthält, wobei die Turbine ferner einen Gaskanal (6) zum Motorende der Welle umfasst, wodurch Spülgas in den Bereich des Fußes der Motorwelle geleitet und verteilt werden kann, um eine Exposition des Motors gegenüber Patientengasen zu verhindern, wobei ein Bund (22, 83) so ausgebildet ist, dass er das Spülgas zur Welle leitet und den Ausfluss von Sauerstoffgas reduziert, um eine minimale Spülgasdurchflussrate aufrechtzuerhalten, **dadurch gekennzeichnet, dass** eine Motorwellen-Oberfläche des Bunds sich in unmittelbarer Nähe zur Motorwelle befindet und in den Bund eingelassene Kreise aufweist, um Gas einzuschließen und eine gasführende Dichtung mit der Motorwelle zu bilden, oder dass eine Laufradseite des Bunds eine Oberfläche mit eingelassenen Kreisen zunehmenden Durchmessers aufweist, um Spülgas einzuschließen und eine Gasdichtung mit der Unterseite des Laufrads zu bilden.

2. Turbine nach Anspruch 1, bei der Spülgas zwischen dem Bund und der Welle in Richtung eines Laufradendes der Welle strömt, um einen minimalen Spülgasstrom aufrechtzuerhalten.

3. Turbine nach einem der vorstehenden Ansprüche, wobei am äußeren Ende des Gaskanals ein Anschluss zum Anschluss einer Spülgasversorgung vorgesehen ist.

4. Turbine nach einem der vorstehenden Ansprüche, bei der der Bund ein separates Bauteil ist, das um die Welle herum am Turbinengehäuse befestigt ist.

5. Turbine nach einem der Ansprüche 1 bis 3, bei der der Bund als Teil des Turbinengehäuses integriert ist.

6. Turbine nach einem der vorstehenden Ansprüche, bei der der Bund so ausgebildet ist, dass das Spülgas anschließend um die Welle herum und unterhalb des Laufrads strömt, um sich mit dem Gas aus einem Atemkreislauf zu vereinigen.

7. Turbine nach einem der vorstehenden Ansprüche, bei der sich der Bund nicht über den gesamten Durchmesser des Laufrads erstreckt oder bei der sich der Bund über den gesamten Durchmesser eines Laufrads erstreckt.

8. Turbine nach einem der vorstehenden Ansprüche, wobei der Bund einen einzelnen Gaskanal oder mehrere Gaskanäle aufweist.

9. Turbine nach einem der vorstehenden Ansprüche, die über ein abnehmbares Laufradgehäuse (73) verfügt, das zur Sterilisation geschickt werden kann, wobei das Turbinengehäuse, der Motor und das Laufrad in der Vorrichtung verbleiben, um die elektrischen Anschlüsse und die Spülgasanschlüsse aufrechtzuerhalten.

10. Turbine nach Anspruch 9, wobei das abnehmbare Laufradgehäuse (73) durch eine Sterilisationsvorrichtung ersetzt werden kann, die Sterilisationsflüssigkeiten, -dämpfe oder - strahlung an das Turbinengehäuse, den Motor und das Laufrad abgibt, um die Teile der Turbine zu sterilisieren, die nach dem Entfernen des Laufradgehäuses in der Vorrichtung verbleiben.

11. Turbine nach einem der vorstehenden Ansprüche, bei der es sich bei dem Spülgas um medizinische Luft und/oder medizinischen Sauerstoff handelt.

12. Anästhesiegerät für Patienten mit einer Turbine gemäß einem der vorstehenden Ansprüche.

13. Patientenbeatmungsgerät mit einer Turbine gemäß einem der Ansprüche 1 bis 11.

## Revendications

1. Turbine destinée à délivrer du gaz à un patient, la turbine comprenant un moteur (7), une roue (3, 71) et un carter comprenant un arbre moteur (85) présentant une extrémité côté moteur, la turbine comprenant en outre un canal de gaz (6) vers l'extrémité côté moteur de l'arbre, moyennant quoi du gaz de balayage peut être délivré à la région de la base de l'arbre moteur et distribué pour empêcher une exposition du moteur aux gaz de patient, dans laquelle un collier (22, 83) est configuré pour délivrer le gaz de balayage à l'arbre et réduire l'écoulement d'oxygène gazeux vers l'extérieur afin de maintenir un débit d'écoulement de gaz de balayage minimal, **caractérisée en ce qu'**une surface côté arbre moteur du collier est à proximité immédiate de l'arbre moteur et présente des évidements circulaires dans le collier pour piéger du gaz et former un joint d'étanchéité de palier à gaz avec l'arbre moteur, ou **en ce qu'**un côté roue du collier présente une surface avec des évidements circulaires d'un diamètre croissant pour piéger le gaz de balayage et former un joint d'étanchéité au gaz avec la face inférieure de la roue.

2. Turbine selon la revendication 1, dans laquelle le gaz de balayage passe entre le collier et l'arbre vers une extrémité côté roue de l'arbre pour maintenir un débit d'écoulement de gaz de balayage minimal.

3. Turbine selon l'une quelconque des revendications précédentes, dans laquelle une connexion peut être réalisée au niveau de l'extrémité externe du canal de gaz pour la connexion d'une alimentation en gaz de balayage.

4. Turbine selon l'une quelconque des revendications précédentes, dans laquelle le collier est un composant séparé qui est fixé au carter de turbine, autour de l'arbre.

5. Turbine selon l'une quelconque des revendications 1 à 3, dans laquelle le collier est intégré en tant que partie du carter de turbine.

6. Turbine selon l'une quelconque des revendications précédentes, dans laquelle le collier est configuré de telle sorte que le gaz de balayage passe ensuite autour de l'arbre et sous la roue pour rejoindre le gaz provenant d'un circuit respiratoire.

7. Turbine selon l'une quelconque des revendications précédentes, dans laquelle le collier ne s'étend pas sur tout le diamètre de la roue, ou dans laquelle le collier s'étend sur tout le diamètre d'une roue.

8. Turbine selon l'une quelconque des revendications précédentes, dans laquelle le collier présente un canal de gaz unique ou de multiples canaux de gaz.

9. Turbine selon l'une quelconque des revendications précédentes, qui présente un carter de roue amovible (73) qui peut être envoyé pour stérilisation et dans laquelle le carter de turbine, le moteur et la roue restent dans le dispositif pour maintenir des connexions électriques et de gaz de balayage.

10. Turbine selon la revendication 9, dans laquelle le carter de roue amovible (73) peut être remplacé par un dispositif de stérilisation qui délivre des fluides, des vapeurs ou un rayonnement de stérilisation au carter de turbine, au moteur et à la roue pour stériliser les parties de la turbine restant dans le dispositif après le retrait du carter de roue.

11. Turbine selon l'une quelconque des revendications précédentes, dans laquelle le gaz de balayage est de l'air médical et/ou de l'oxygène médical.

12. Dispositif d'anesthésie de patient présentant une turbine selon l'une quelconque des revendications précédentes.

13. Ventilateur de patient présentant une turbine selon l'une quelconque des revendications 1 à 11.
